# EUROPEAN PATENT APPLICATION

(11) **EP 2 799 029 A1**
(43) Date of publication of application: **05.11.2014**
(21) Application number: 14164302.3
(22) Date of filing: 10.04.2014
(51) Int. Cl.: A61B 19/00

(54) **Computer-assisted guidance of a surgical instrument during diagnostic or therapeutic operations**

(30) Priority: 30.04.2013 IT TO20130349
(71) Applicant: MASMEC S.p.A., Modugno (IT)
(72) Inventor: Larizza, Pietro, 70026 Modugno (IT); Vinci, Angelo Michele, 70026 Modugno (IT)
(74) Representative: Bongiovanni, Simone

(57) **Abstract**

A computer-assisted system (100) for guiding a surgical instrument (25) in the body of a patient wherein an electronic unit is configured to: import a plurality of two-dimensional images I1, I2, ... Ii,... In of an internal portion of the human body of the patient; process the imported images in order to define a reconstructed three-dimensional image that represents an anatomical volume of interest; perform scans of a portion of the patient's body by means of a calibrator marker (24); compare one or more scans with the curves defined on the three-dimensional image to detect matches; if a least one match is detected, the reference system of the reconstructed two-dimensional image is aligned with the reference system of a location sensor (20).

## Description

### TECHNICAL FIELD OF INVENTION

The present invention relates to the computer-assisted guidance of a surgical instrument in the body of a patient, in particular for assisting medical personnel during diagnostic and/or therapeutic operations.

### STATE OF THE ART

As is known, operations for biopsy, thermal ablation, removal of injured tissue, etc. are currently performed with the aid of a surgical instrument (operating tool) able to reach, via percutaneous access, an area affected by a pathology (often indicated as the target area). The operation of introducing and subsequently reaching the target area with a surgical instrument may be aided by guidance or navigation systems, of a virtual type, based on images of the target and the areas surrounding of the target so as to plan and perform the percutaneous operation in the least invasive manner. For example, these images can, be acquired in real time by means of an X-ray (TC) or magnetic resonance (RM) apparatus.

However, there are various limitations in using images in real time for helping the surgeon in performing the operation. For example, the use of computerized tomography does not allow acquiring images in real time during the therapeutic operation due to the practical impossibility of manipulating the surgical instrument during the phase of TC scanning of the patient on whom the operation is being performed and due to safety reasons concerning the doctor performing the operation, given the potential harmfulness of ionizing radiation.

Known systems for image-assisted therapeutic operations are based on images acquired via TC and contemplate the use of images acquired prior to the operation for the purposes of creating a three-dimensional reconstruction in virtual reality of the area of the patient's body to be operated on. A three-dimensional representation of the surgical instrument is then overlaid on this three-dimensional reconstruction during the various phases of the operation. It is obvious that for this purpose, the surgical instrument must also be fitted with appropriate sensors in such a way that its three-dimensional representation may be inserted in the three-dimensional reconstruction of the area of the human body to be operated on.

Patent EP09425116, by the same applicant, illustrates a computer-aided system for guiding a surgical instrument in the body of a patient comprising a first marker device configured to be arranged so as to be integral with a region of a patient's body and including first and second markers having a given reciprocal arrangement; a second marker configured to be coupled to the surgical instrument and including third marker elements; a locating sensor configured to localize the second and third marker elements in a first reference system; and a processing unit configured to acquire at least one tomography image of the region of the patient's body comprising the first marker elements in un second reference system different from the first, acquire the position of the second and third marker elements in the first reference system, and determine the position of said third marker elements in the second reference system on the basis of a correlation between the first and the second reference systems.

### SUBJECT AND ABSTRACT OF THE INVENTION

The object of the present invention is that of improving the system described in aforementioned patent, enabling percutaneous navigation to be performed without the aid of a TC or RM apparatus in the operating room or theatre. In fact, operating theatres do not currently have imaging systems such as TC or RM able to help the surgeon concomitantly with the operation. An assumption of foregoing patent EP09425116 was that of being able to have a sensor, known as a patient sensor, on the patient's body and close to the operating region that is able to follow the position of the patient in real time and correlate it with the position of the operating instrument, this also provided with an opportune sensor. In this way, the adjustment or calibration operations, or rather the operations that make the domain of the spatial coordinates concerning the sensorized objects (patient sensor and operating instrument sensor) and the domain of the image coordinates of the images displayed on the viewer perfectly superimposed, may be carried out once the patient sensor is positioned on the patient's body prior to the tomographic scan. This implies a) the proximity of the tomographic apparatus to the operating room; b) the impossibility of using scans that may already be available of the same patient but do not indicate the presence of the patient sensor on the body.

According to the present invention, a computer-assisted system and a method are provided for guiding a surgical instrument in the body of a patient that overcome the limitations of foregoing patent EP09425116 and are as defined in the appended claims.

### BRIEF DESCRIPTION OF DRAWINGS

- Figure 1 schematically shows a computer-aided system for guiding a surgical instrument in the body of a patient according to a first embodiment of the present invention making use of an infrared ray tracing system with a contact calibrator element;
- Figure 2 schematically shows a computer-aided system for guiding a surgical instrument in the body of a patient according to a second embodiment of the present invention making use of an infrared ray tracing system with a non-contact calibrator element;

- Figure 3 schematically shows a computer-aided system for guiding a surgical instrument in the body of a patient according to a third embodiment of the present invention making use of an electromagnetic wave tracing system with a contact calibrator element;
- Figure 4 schematically illustrates a computer-aided system for guiding a surgical instrument in the body of a patient according to a fourth embodiment of the present invention making use of an electromagnetic wave tracing system with a non-contact calibrator element; and
- Figure 5 illustrates a series of operations performed by the system according to the present invention.

### DESCRIPTION

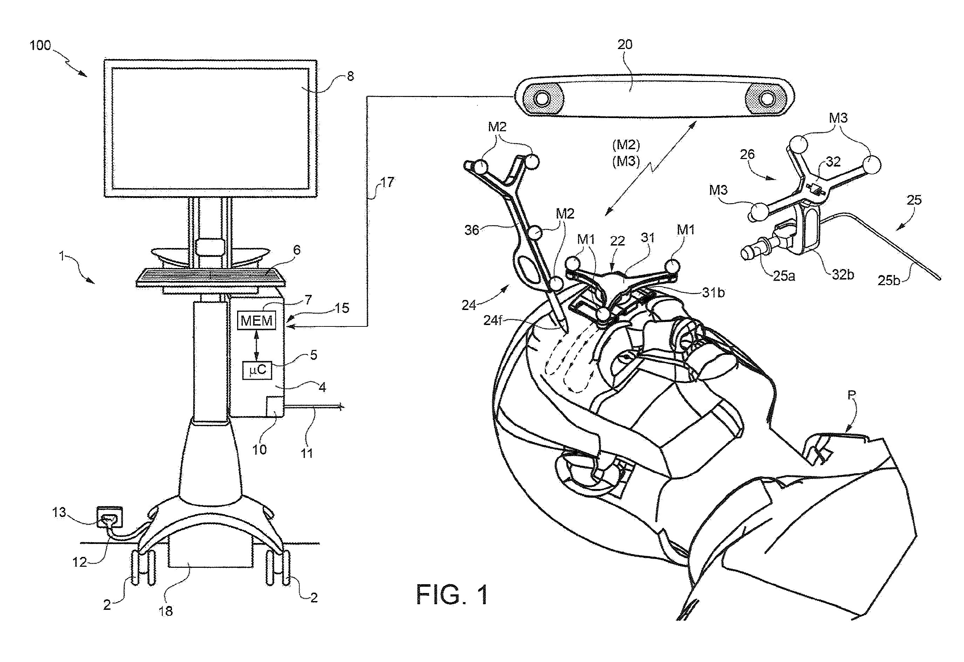

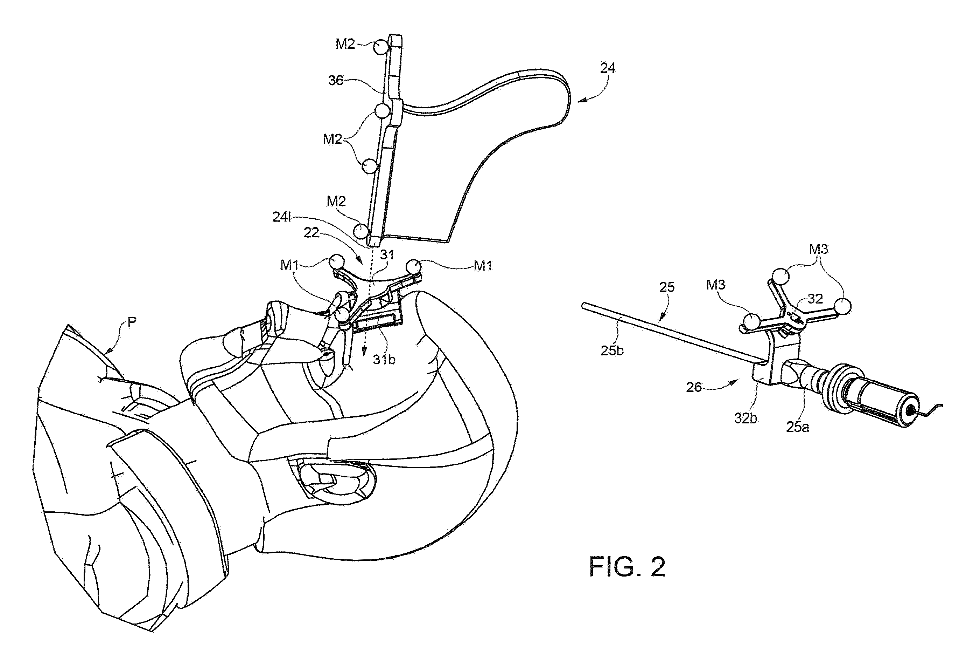

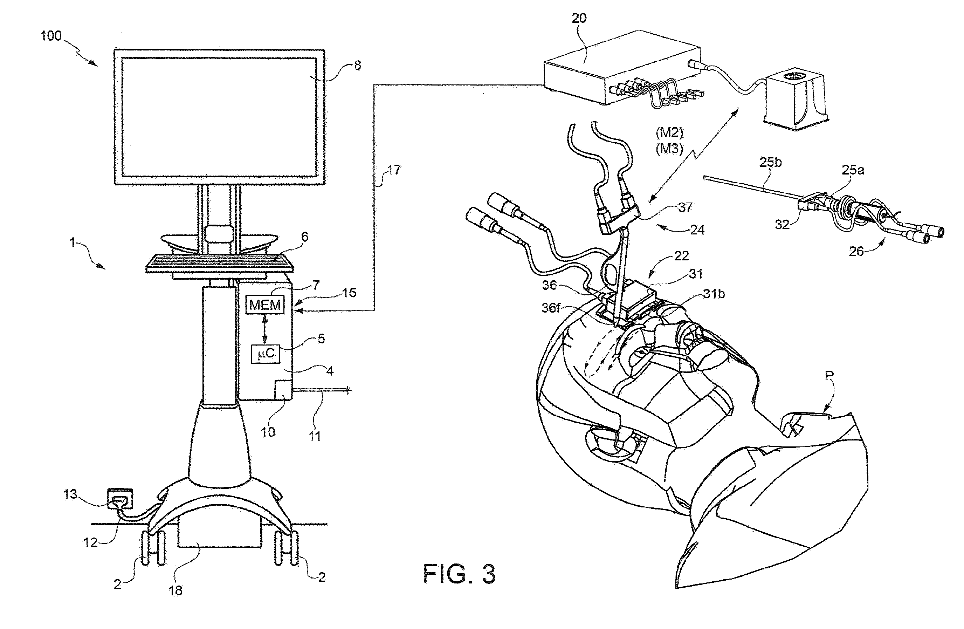

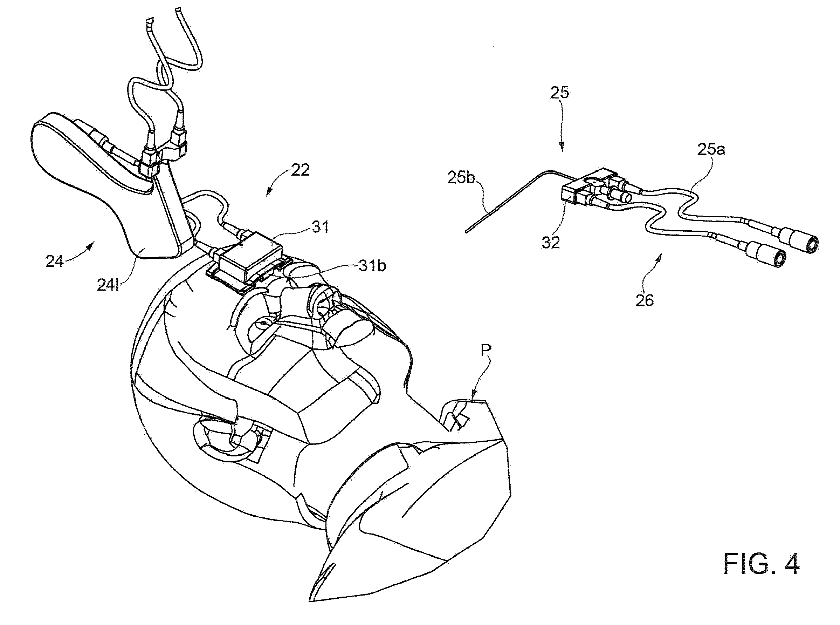

Figures 1 and 2 show a system 100 according to the present invention comprising an instrumental station 1; an infrared (Figure 1) or electromagnet (Figure 3) tracing sensor 20, of known type, configured to cooperate with the instrumental station 1; a patient marker 22, provided with infrared (Figures 1 and 2) or electromagnetic (Figures 3 or 4) marker elements M1 and configured to be arranged on a portion of the body (in the example, on the forehead) of a patient P and to cooperate with the tracing sensor 20 (as better described hereinafter); a calibrator marker 24, provided with a plurality of second infrared (Figures 1 and 2) or electromagnetic (Figures 3 and 4) marker elements M2 and configured to be used as a calibration tracer in the contact version (Figures 1 and 3) and in the non-contact version (Figures 2 and 4) housing a laser-beam distance meter integral therewith (illustrated hereinafter); and an instrument marker 26, provided with a plurality of infrared (Figures 1 and 2) or electromagnetic (Figures 3 and 4) third marker elements M3 and configured to be coupled to a surgical instrument 25 to cooperate with the infrared/electromagnetic tracing sensor 20 (as better described hereinafter).

In particular, the surgical instrument 25 (an operating tool in the case of Figure 1 and an endoscopic tool in the case of Figures 2 and 3) comprises a grippable proximal portion 25a and an operative distal portion 25b; the instrument marker 26 is coupled to an intermediate portion, leaving the distal portion 25b free. It should be noted that, for greater clarity of representation, the relative sizes of the elements shown in Figures 1, 2, 3 and 4 are not proportional to each other.

The instrumental station 1 (Figures 1 and 3) comprises a processing unit 4, for example a computer of known type provided with a microcontroller 5 and memory 7, connected to each other; a data input user interface 6, for example including a keyboard and mouse; a display interface 8, for example a high-resolution LCD monitor; and a network interface 10, configured to support a private and/or public network connection 11 (for example, an Ethernet network).

The instrumental station 1 further comprises a power connection 12, configured to provide the instrumental station 1 with electrical power by means of a wall-mounted power socket 13; a tracing input 15, configured to support a connection 17 (indifferently of the wireless or wired type) between the instrumental station 1 and the infrared or electromagnetic tracing sensor 20; and an energy storage unit 18, for example a battery, connected to the wall socket 13 by the power connection 12 and configured to temporarily power the instrumental station 1 in the event of an outage in the power provided by the wall socket 13.

According to the embodiment shown, the processing unit 4 is a personal computer (PC), comprising an external protective casing housed on a shelf of the instrumental station 1 and integral with the instrumental station 1 during any movement of the latter on the wheels 2.

In order to ensure that the coordinates belonging to the surgical instrument 25 and acquired by the infrared/electromagnetic tracing system 20 always refer the body of the patient P, the patient marker 22 is placed in contact with the patient so as to be integral therewith (in the modes illustrated hereinafter - please refer to Figure 1 and Figure 3) in a region of the patient (externally) close to the operating region.

According to the embodiment shown, the infrared version of the patient marker 22 in Figure 1 and 2 comprises a body 31, for example made of a plastic material or, more in general, a material transparent to the image acquisition system employed. The body 31 is substantially Y-shaped and comprises three arms at the ends of which the spherically shaped marker elements M1 are arranged, the latter being detectable by means of the infrared tracing sensor 20. The body 31 has a base portion 31b that is shaped to be stably rested on the patient's body (in the example, it is a deformable flat parallelepiped portion placed on the forehead of the patient P).

In the electromagnetic version in Figures 3 and 4, the patient marker 22 comprises a parallelepipedal body 31, for example made of a plastic material or, more in general, a material transparent to the image acquisition system employed. The body 31 houses a parallelepipedal or cylindrical electromagnetic sensor of known type (not shown) that may be detected by the electromagnetic tracing sensor 20. The body 31 has a base portion 31b that is shaped to be stably rested on the patient's body (in the example, it is a deformable flat parallelepiped portion placed on the forehead of the patient P).

In the infrared version in Figures 1 and 2, the instrument marker 26, in particular utilizable with a surgical instrument, comprises a Y-shaped body 32 quite similar to the patient marker but of different size and comprising three arms on which three markers M3 are arranged.

The body 31 comprises a base portion 32b that is stably fastened to a central portion of the surgical instrument 25.

In the electromagnetic version in Figures 3 and 4, the instrument marker 26 comprises a body 32, for example made of a plastic material or, more in general, a material transparent to the image acquisition system employed. The body 32 houses two parallelepipedal or cylindrical electromagnetic sensors (not shown and of known type) that may be detected by the electromagnetic tracing sensor 20.

In the infrared version in Figure 1 and 2, the calibrator marker 24 comprises a Y-shaped body 36 comprising three arms on which four marker elements M2 are arranged. In the example shown, two marker elements M2 are arranged at the ends of the respective shorter arms of the Y-shaped body and two marker elements M2 are arranged in line on the central arm of the Y-shaped element 36. In the contact version (Figure 1), the calibrator marker 24 is provided with a tapered end portion 24f configured to slide easily over the patient's body; in the non-contact version (Figure 2), it is provided with a laser pointer 241 that functions as a distance meter to detect the distance between the calibrator marker and the scanned portion of the patient P. This laser-beam distance meter is capable of transmitting the measured distance data to the processing unit 10. By scanning the portion of the human body with the non-contact calibrator marker 24, it is possible to measure the distance between the marker 24 (the position of the marker in space is detected by the infrared or electromagnetic location sensor 20) and consequently reconstruct, by means of known techniques, the profile/section of the scanned portion of the human body.

In the electromagnetic version in Figure 3 and 4, the calibrator marker 24 comprises a grippable styliform body 36 that houses a parallelepipedal or cylindrical electromagnetic sensor 37. In the contact version (Figure 3), the calibrator marker 24 is provided with a tapered end portion 36f of the styliform body configured to slide easily over the body of the patient P; in the non-contact version (Figure 4), it is provided with a laser pointer 241 that functions as a distance meter. This laser-beam distance meter is capable of transmitting the measured distance data to the processing unit 10.

In the infrared ray version, each marker element M1, M2 and M3 is made of an infrared-reflective material, so that it may be detected by the infrared tracing sensor 20. The marker elements M1, M2 and M3 are also arranged with respect to each other in such a way that both a rotation and a displacement of the patient marker, instrument marker (and therefore of the surgical instrument 25 with which the instrument marker M3 is integral) and calibrator marker devices are immediately detectable. In the case where the non-contact calibrator marker is used, in addition to the detected position provided by the marker elements M2, the processing unit also takes into account the distance measured by the laser pointer 241 integral with the non-contact calibrator marker.

Likewise, in the electromagnetic version, the above-described markers perform the role of detectors for both the rotation and the displacement of the related elements integral with them.

The following description is therefore indifferent to whether the version using infrared rays or the one using electromagnetic waves is considered as the tracing system for the attitudes of the elements M1 M2 and M3.

The two-dimensional images, acquired by the computerized tomography device 21, also at times prior to the operation, are supplied to the processing unit 4, which creates a three-dimensional representation of the internal organs by means of known techniques that are already widely used for this purpose.

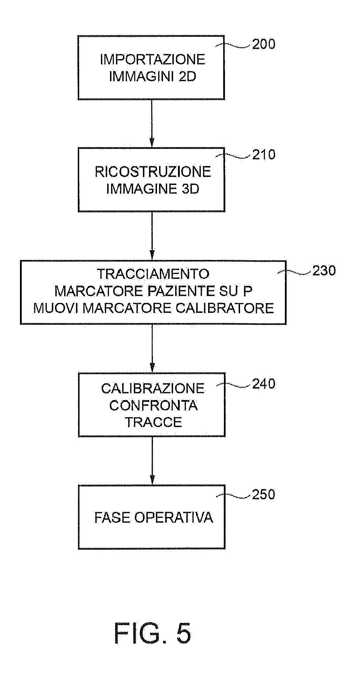

According to the present invention (Figure 5), a calibration step is performed in which the processing unit 4 carries out an acquisition step (block 200) wherein a plurality of two-dimensional images I1, I2, ... Ii,... In of an internal portion of the human body of a patient are imported (for example two-dimensional images of the braincase). These images of the patient have been acquired beforehand.

In particular, DICOM two-dimensional X-ray images or two-dimensional images obtained by magnetic resonance may be imported.

The importation of these images may be achieved in a known manner by using a computer medium (CD, mass storage, etc.) that may be inserted in the processing unit 4 or by connecting the computer medium to the processing unit 4 via a network cable.

Upon completing the three-dimensional volumetric reconstruction step, the imported images may be displayed on the display interface 8 in different ways.

The electronic unit 4 then performs (block 210, following block 200) a three-dimensional reconstruction process based on the imported images in order to define a three-dimensional image that represents an anatomical volume of interest. The points (pixels) of the three-dimensional image refer to its own image reference system. The three-dimensional reconstruction process is carried out using known techniques and will therefore not be described any further. In this case as well, the dimensional image may be displayed on the display interface 8 in different ways (axial, sagittal and rotational views).

A tracing step is then performed (block 230, following block 210), during which the patient marker 22 (infrared or electromagnetic type) is placed in contact with a portion of the patient's body (in the example, the patient marker 22 is positioned on the patient's forehead) in such a way that it assumes a stable position on the patient P and the end portion 24f/36f of the calibrator marker 24 - in the case where the contact mode described with reference to Figures 1 and 3 is used - is placed in contact with the patient's skin, making it slide by hand over the skin in such a way that the infrared marker elements M2 (or the electromagnetic marker elements) follow the profile in space of the portion of the patient.

The infrared/electromagnetic tracing sensor 20 is activated to capture the trajectories made by the calibrator marker 24, constituted by paths traced on a portion of the patient's forehead. The scans are detected by the tracing sensor 20 and stored in the processing unit.

When scanning the portion of the human body by means of the non-contact calibrator marker 24, it is possible to detect the distance between the marker 24 (the position of the marker in space is detected by the infrared or electromagnetic location sensor 20, which detects elements opaque to infrared/electromagnetic radiation) and then reconstruct, by means of known techniques, the profile/section of the scanned portion of the human body.

The presence of the patient marker 22 provided with first marker elements M1 (infrared-reflecting or electromagnetic) enables defining the position of the calibrator marker 24 with respect to the patient marker 22 (and therefore to the patient).

When the tracing step is completed, a calibration procedure is launched (block 240, following block 230) during which the previously performed scans are compared (for example, by using a correlation algorithm) with similar curves on the reconstructed three-dimensional image.

If a match is found between a scan and a curve of the three-dimensional image (i.e. if a match is found between the physically scanned portion and a corresponding portion of the tree-dimensional image) it is possible to align the image reference system with the reference system of the tracing sensor 20 thanks to the presence of the patient marker 22. The realignment operation is carried out using roto-translation algorithms known in the literature.

Upon completion of the operations performed in block 230, the reference system of the infrared detection system 20 and that of the three-dimensional image reconstructed from the imported images are aligned.

An operative step (block 250, following block 240) is then performed in which the three-dimensional image, reconstructed in block 210 and having a reference system coincident with the reference system of the infrared/electromagnetic tracing sensor 20, is displayed and used by the system 1.

The processing unit 4 now performs virtual navigation according to the modes described in patent EP09425116 using the aforesaid correctly positioned three-dimensional image as a three-dimensional model.

In this way, the processing unit 4 enables displaying the following on the display unit 8:
- a three-dimensional representation of the region of the body generated on the basis of the tomographic image acquired by the apparatus 21 superimposed on the reconstructed and correctly positioned three-dimensional image; and
- a three-dimensional representation of the operational portion 25b of the surgical instrument 25, graphically superimposed on the three-dimensional representation of the region of the patient's body, on the basis of the position of third marker elements M3 in the second reference system and the numerical model of the surgical instrument stored in the memory 7.

In this way, a system is provided that is easy to use by a doctor oriented to minimally invasive operations in the operating theatre.

During the phases of surgery, a three-dimensional model of the area of the patient being operated on is shown on the display interface 8, i.e. by displaying internal organs and tissues. As said, this three-dimensional model is generated by the processing unit 4.

In this way, it is possible to render the internal position of tissues according to the image obtained with a computerized tomography scan coherent with the current position during the operation.

The display interface 8 also shows a three-dimensional model of the surgical instrument used superimposed on the three-dimensional model of the area of the patient being operated on. The type of surgical instrument 25 to be displayed may be chosen by the doctor performing the operation from a plurality of possible models, previously created and stored in the memory 7 of the processing unit 4. Alternatively, the choice of the type of surgical instrument 25 may be performed automatically by the processing unit 4 based on the arrangement of the third marker elements M3 of the instrument marker 26 (as previously described).

The doctor manipulating the surgical instrument 25 fitted with the instrument marker 26 is guided by the images displayed on the display interface 8 throughout the entire operation. The trajectory of the surgical instrument 25 is calculated with the aid of artificial-intelligence algorithms so that all of the area involved is treated with the minimum number of insertions, ensuring total coverage without striking vital and/or obstructing organs. These organs are identified via the three-dimensional model shown on the display interface 8. The position of the surgical instrument 25 is inserted in the three-dimensional model thanks to the measurement that the tracing sensor 20 makes on the spatial coordinates of the instrument marker 26. In practice, it is possible to acquire up to three Cartesian coordinates (X, Y, Z) and up to three polar coordinates (α, β, γ). This results in the real movements of the surgical instrument 25 being transferred in real time, via the connection 17, to the processing unit 4 to obtain an updated representation of the state of the operation on the display interface 8. Alternatively, it is possible to simulate, in a similar manner to that previously described, the a priori trajectory of the surgical instrument 25 in order to establish the optimal path once the target to be treated is identified. Once the optimal trajectory is identified, the insertion is performed by the doctor manipulating the surgical instrument 25 fitted with the instrument marker 26.

## Claims

1. A computer-assisted system (100) for guiding a surgical instrument (25) in the body of a patient comprising:
• a first patient marker device (22) configured to be arranged integral with a region of the body of a patient to be treated by means of said surgical instrument (25) and including first marker elements (M1);
• a second calibrator marker device (24) including second marker elements (M2);
• a third instrument marker device (26) configured to be coupled with said surgical instrument (25) and including third marker elements (M3);
• a location sensor (20) configured to locate said first, second and third marker elements (M1, M2, M3); and
• a processing unit (4) connected to the location sensor (20), **characterized in that** said processing unit (4) comprises:
a) acquisition means (200) via which a plurality of two-dimensional images I1, I2, ... Ii,... In of an internal portion of the human body of the patient are imported;
b) reconstruction means (210) adapted to process the imported images in order to define a three-dimensional image that represents an anatomical volume of interest; the points of the three-dimensional image refer to its own image reference system;
c) tracing means (230) adapted to activate the location sensor (20) to detect the position of the second marker elements (M2) in space while the calibrator marker (24) is moved on said region of the patient with a portion of the location sensor that follows the profile of said region, performing at least one scan of the profile; said tracing means being adapted to detect and store the scans subsequently performed; said tracing means being furthermore adapted to establish the position of said first patient marker device with respect to a reference system of the location sensor (20) ;
d) calibration means (240) configured to compare the previously performed scans, for example using a correlation algorithm, with similar curves on the reconstructed three-dimensional image; if a match is found between at least one performed scan and a curve of the three-dimensional image, said match being indicative of a match between the scanned portion and a corresponding portion of the three-dimensional image, said calibration means are configured to activate roto-translation means configured to align the image reference system with said reference system of the location sensor (20).

2. A system according to claim 1, wherein the location sensor (20) is of the infrared type and said first, second and third marker elements (M1, M2, M3) are opaque to the infrared radiation.

3. A system according to claim 1 or 2, wherein said first, second and third marker elements (M1, M2, M3) comprise a plurality of bodies opaque to the infrared radiation having reciprocal pre-determined positions.

4. A system according to any of the preceding claims wherein said first patient marker device (22) comprises a main body (31) adapted to support first marker elements (M1) and a base portion (31b) that is shaped to be stably rested on the body of the patient (P).

5. A system according to claim 4, wherein said main body (31) is Y-shaped and comprises three arms at the end of each of which a respective first marker element (M1) is arranged.

6. A system according to claim 1, wherein the location sensor (20) is of the electromagnetic type and said first, second and third marker elements (M1, M2, M3) may be detected by electromagnetic radiation.

7. A system according to any one of the preceding claims, wherein said second calibrator marker device (24) is of the contact type and comprises a grippable main body (36) bearing said second marker elements provided with an end portion (24f, 36f) configured to be arranged in contact with and slide over the body of the patient.

8. A system according to any one of claims 1 to 7, wherein said second calibrator marker device (24) is of the non-contact type and is provided with a distance meter, for example of the laser type, to identify the distance between said calibration marker (24) and the scanned portion of patient (P); said calibration marker is configured to transmit the distance measured to the processing unit (4) for the reconstruction of said profile.

9. A method for guiding a surgical instrument (25) in the body of a patient where:
• a first patient marker device (22) is configured to be arranged integral with a region of the body of a patient to be treated and includes first marker elements (M1);
• a second calibration marker device (24) includes second marker elements (M2);
• a third instrument marker device (26) is configured to be coupled with said surgical instrument (25) and includes third marker elements (M3);
• a location sensor (20) is configured to locate said first, second and third marker elements (M1, M2, M3); and **characterized in that** it comprises the following steps:
a) import (200) a plurality of two-dimensional images I1, I2, ... Ii,... In of an internal portion of the human body of the patient;
b) process (210) the imported images in order to define a three-dimensional image that represents an anatomical volume of interest; the points of the three-dimensional image refer to its own image reference system;
c) activate (230) the location sensor (20) to detect the position of the second marker elements (M2) in space while the calibrator marker (24) is moved with respect to said region of the patient to follow the profile of said region in space, performing at least one scan of the profile;
d) establish the position of said first patient marker device with respect to a reference system of the location sensor (20);
e) compare (240) the previously performed scans, for example using a correlation algorithm, with similar curves on the reconstructed three-dimensional image; if a match is found between at least one performed scan and a curve of the three-dimensional image, said match being indicative of a match between the scanned portion and a corresponding portion of the three-dimensional image, a roto-translation algorithm is used to align the image reference system with said reference system of the location sensor (20).

10. A method according to claim 9, wherein said movement phase is performed with contact, sliding an end portion (24f, 26f) of the second calibrator marker device (24) over the scanned portion.

11. A method according to claim 9, wherein said movement phase is performed without contact, moving the second calibrator marker device (24) with respect to the scanned portion and measuring the distance between the calibrator marker device (24) and the scanned portion; said method comprising the step of reconstructing the profile/section of the scanned portion by means of the distance measured.
